# EUROPEAN PATENT SPECIFICATION

(11) **EP 1 076 100 B1**
(45) Date of publication and mention of the grant of the patent: **09.06.2010**
(21) Application number: 99917181.2
(22) Date of filing: 26.04.1999
(51) Int. Cl.: C13K 13/00, C07H 3/02, C12P 7/18

(54) **PROCESS FOR PRODUCING L-ARABINOSE BY ACID HYDROLYSIS METHOD**
VERFAHREN ZUR HERSTELLUNG VON L-ARABINOSE DURCH SAURE HYDROLYSE
PROCEDE DE PRODUCTION DE L-ARABINOSE PAR HYDROLYSE ACIDE

(30) Priority: 01.05.1998 JP 13748598
(43) Date of publication of application: 14.02.2001
(73) Proprietor: Sanwa Kosan Kabushiki Kaisha, Kashihara-shi, Nara 634-0834 (JP)
(72) Inventor: HIZUKURI, Susumu, Kagoshima 892-0811 (JP); ABE, Jun'ichi, Kagoshima 891-0145 (JP); OHSAKI, Shigemitsu, Nara 634-0008 (JP); SUETAKE, Shuichi, Kitakatsuragi-gun, Nara 635-0831 (JP); SHIBANUMA, Kiyoshi, Nara 634-0834 (JP)
(74) Representative: Gaunt, Robert John
(86) International application number: PCT/JP1999/002240
(87) International publication number: WO 1999/057326

(56) References cited:
- JP-A- 1 312 997
- JP-A- 9 299 093
- JP-A- 58 039 695
- JP-A- 59 159 791
- US-A- 4 772 334
- US-A- 4 816 078
- US-A- 5 846 794
- CHEMICAL ABSTRACTS, vol. 71, no. 16, 20 October 1969 (1969-10-20) Columbus, Ohio, US; abstract no. 72113c, STARICHKOVA, V. E. ET AL: "Interaction of hydrochloric acid solutions with cornstalk and millet hull hemicelluloses" XP001005319 & KHIM. DREV. , vol. 3, 1969, page 27-32
- CHEMICAL ABSTRACTS, vol. 70, no. 20, 19 May 1969 (1969-05-19) Columbus, Ohio, US; abstract no. 88963v, DUDKIN, M. S. ET AL: "Hydrolysis of hemicelluloses from a mixture of corn stalks and straw by dilute sulfuric acid solutions" XP001005280 & ZH. PRIKL. KHIM. (LENINGRAD), vol. 41(12), 1968, page 2711-2717
- DATABASE WPI Section Ch, Week 199850 Derwent Publications Ltd., London, GB; Class D16, AN 1998-592628 XP002170349 & RU 2 109 059 C (BLINKOV S D), 20 April 1998 (1998-04-20) & WO 99 23260 A (BLINKOV S D) 14 May 1999 (1999-05-14)
- TSCHIERSCH, B. ET AL: "Zur Darstellung von L-(+)Arabinose aus Zuckerrüben" PHARMAZIE, vol. 36(2), 1981, page 159-160 XP000986251
- SCHIBANUMA ET AL.: "Partial Acid Hydrolysis of Corn Fiber for the Production of L-Arabinose" J. APPL. GLYCOSCI., vol. 46, no. 3, 1999, page 249-256 XP000996422
- SAHA ET AL: "Production of L-arabitol from L-arabinose by Candida entomaea and Picia guilliermondii" APPLIED MICROBIOLOGY AND BIOTECHNOLOGY, vol. 45, no. 3, 1996, pages 299-306, XP000934273

## Description

### Technical Field of the Invention

The present invention relates to a process for the manufacture of L-arabinose which is very useful in view of physiological function in an efficient manner and at low cost from the fiber derived from vegetables obtained as a by-product in the manufacturing steps in various milling factories and starch factories.

### Background Art

A part of the fiber derived from vegetables obtained as a by-product in the manufacturing steps in various milling factories and starch factories is used as a material for xylose and food fiber but most of it is used as a feed for poultry and a fuel or becomes an industrial waste and, therefore, there has been a demand for its effective utilization.

Such a fiber is composed of cellulose (where D-glucose is a constituting saccharide), L-arabinose, D-xylose, L-fucose, D-galactose, D-mannose, L-rhamnose, D-galacturonic acid and D-glucuronic acid as constituting saccharides and, in addition thereto, hemicellulose including ferulic acid, polysaccharide such as pectin substances, lignin which is a polymer of phenylpropane, etc. There is a report that, among them, arabinoxylan composed of arabinose and xylose is abundant as a hemicellulose component (Handbook of Dietary Fiber, Marcel Dekker, Inc. 1987).

With regard to L-arabinose, usefulness in view of its physiological function has been reported and, if L-arabinose can be supplied in the market in large quantities and at low cost, that would be expected for human pleasant food life and maintenance of good health and also as a food for prevention of diseases such as diabetes mellitus caused by hyperglycemia. Unfortunately, however, that has not been commercially available as food and pharmaceuticals yet.

As examples for its useful physiological function, usefulness as a preventive and therapeutic agent for diseases accompanied by hyperglycemia due to an α-glucosidase inhibiting effect and also as a health food is disclosed in the Japanese Patent Laid-Open No. 65080/1994 and usefulness as a suppressor for accumulation of body fat is disclosed in the Japanese Patent Laid-Open No. 242551/1995. As such, its usefulness as an edible food and also in view of physiological function can be expected as well.

With regard to the effective utilization of fiber, there is a disclosure, for example, in the Japanese Patent Laid-Open No. 299093/1997 for a method where sugar beet arabinan in sugar beet pulp is hydrolyzed with an enzyme to manufacture arabinose. Content of arabinose in dry sugar beet pulp is 12-13% by weight while, in Example 1, total arabinan is extracted by heating the sugar beet pulp with calcium hydroxide at 90-100°C for 12 hours whereupon 55 g of total arabinan is prepared using 3.7 kg of sugar beet pulp and, therefore, the extracting rate of total arabinan from sugar beet pulp is calculated as 1.49%. In addition, in Decomposing Example 1 for arabinan, total arabinan (arabinose purity: 72%) is hydrolyzed with an enzyme whereupon 83.3% of total arabinose is liberated and obtained and, therefore, the yield of arabinose which is liberated and obtained from the sugar beet pulp is calculated as 0.89% (1.49 x 0.72 x 0.833). This corresponds to only 7.4-6.8% of the arabinose content in the sugar beet pulp.

It is clear from the above that the yield of arabinose from the material is very low in a process for the manufacture of arabinose by an enzymatic method and, accordingly, such a method is not practical as a commercial manufacturing method where a large-scale production at a low cost is the object.

In the Japanese Patent Laid-Open No. 312997/1989, there is a disclosure for a combination method for the manufacture of selected monosaccharides from envelopes of corn grains by hydrolysis and its content relates to a combined method where hemicellulose contained in the envelopes of corn grains is subjected to an acidic hydrolysis using a strong acid and/or the cellulose component is hydrolyzed with an enzyme to manufacture a mixed solution of D-xylose, L-arabinose and D-glucose and/or a monosaccharide of a D-glucose solution. Thus, it fundamentally provides a method of selective hydrolysis for the two sections - a hexose section of D-glucose component and a pentose section containing D-xylose, L-arabinose and D-glucose - and, in view of an object of commercial manufacture of L-arabinose, the L-arabinose content in the monosaccharide mixture is too low. Thus, for example, in the case of Example 1 where an acidic hydrolysis at 85°C, yields of the hydrolysates in terms of % by weight are 24.9 for D-glucose, 13.6 for D-xylose and 9.0 for L-arabinose wherefrom the L-arabinose content in the monosaccharide is calculated as low as 18.9% and there is a disadvantage that such a method is not practical.

US-A-4772334 discloses the hydrolysis of gum arabic with a mineral acid of 0.1 to 0.6N in order to produce a mixture of L-arabinose, L-rhamnose and D-galactose in a ratio of 2:1:1. Chemical Abstracts, Vol. 70, No. 20, 19 May 1969, Columbus, Ohio, US; Abstract No. 88963v, Dudkin, M.S. et al. "Hydrolysis of hemicelluloses from a mixture of corn stalks and straw by dilute sulfuric acid solutions" XP001005280 describes a hydrolysis with sulfuric acid of 0.2N; the process results in a mixture of L-arabinose, D-xylose, D-glucose and D-galactose in a ratio of 2:1:1. CSSR Patent No. 153378 (1975) discloses the hydrolysis of extracted sugar beet pulp with a 0.1 to 6.0% (0.02N to 1.2N) concentration of sulfuric acid at a temperature over or below 100°C for 10 minutes to 3 hours and the recovery of L-arabinose, pectin and cellulose.

### Problems to be Solved by the Invention

Thus, in the conventional methods, an enzymatic method is advantageous with an object of specific liberation and production of L-arabinose in vegetable fiber as a monosaccharide in a high purity but such a method has a disadvantage that the yield is very low and is not practical in view of the productivity. On the other hand, when importance is paid on the yield, an acidic hydrolysis using a strong acid is advantageous but there is no specificity in the acidic hydrolysis but, in addition to the necessary L-arabinose, unnecessary monosaccharides are also hydrolyzed, liberated and produced whereupon, as a result, there is a disadvantage that purity of L-arabinose extremely lowers making the manufacture of L-arabinose difficult.

The present invention has been created for solving such disadvantages in the prior art and its object is to provide a process for the manufacture of L-arabinose which is excellent in view of physiological function and is safe as a food from a by-product where the vegetable fiber containing L-arabinose as a part of the constituting saccharides is subjected to an acidic hydrolysis under such a mild condition that L-arabinose is specifically obtained in high purity, good efficiency and high yield and that the secondary decomposition reaction hardly takes place.

### Means for Solving the Problems

The present inventors have paid their attention to the fact that L-arabinose is present as a furanose type in vegetable fiber and mostly at the non-reducing terminals (e.g., L. Saulnier, et al., Carbohydrate Polymers, 26 (1995), 279-287) and to the possibility that it is easily liberated by means of decomposition with an acid and have carried out an intensive study on an acidic hydrolysis method for liberation and production of L-arabinose in a specific manner in high yield and purity from vegetable fiber containing L-arabinose as a part of the constituting saccharides where the commonly-used previous extraction of hemicellulose such as arabinan and arabinoxylan with an alkali is eliminated. As a result, they have found that, when conditions for acidic hydrolysis and stage of completion of the acidic hydrolysis are specified, the problems can be solved whereupon the present invention has been achieved.

Thus, the present invention relates to a process for the manufacture of L-arabinose, characterized in that vegetable fiber which is a by-product in the manufacture of corn starch, such as envelopes of corn grains, axis of ear of corn, wheat bran, barley bran, oat bran, rye bran, rice bran or defatted rice bran, is contacted with sulfuric acid, hydrochloric acid or oxalic acid, an acidic hydrolysis is carried out under conditions such that i) the concentration of acid is from 0.01 N to 0.15N for sulfuric acid or hydrochloric acid, and from 0.01 N to 0.50N for oxalic acid, and ii) the proportion of L-arabinose in the total amount of the acid-hydrolyzed monosaccharides is 50% or more, whereby L-arabinose contained in the vegetable fiber is selectively produced.

In a preferred embodiment of the present invention, the vegetable fiber contains 10% or more of at least L-arabinose as a part of the constituting saccharides on the basis of the dried vegetable fiber.

Preferred conditions for the acidic hydrolysis according to the present invention are that the concentration of the acid used is 0.01N to 0.15N; the concentration of the substance to be hydrolyzed (vegetable fiber) is 3% (w/w) to 20% (w/w); and the temperature during the acidic hydrolysis is 80°C to 150°C or, preferably, 96°C to 145°C.

In a preferred embodiment of the present invention, the acidic hydrolysis is carried out under such conditions that the total amount of the saccharides decomposed and eluted during the acidic hydrolysis is 30% or more on the basis of the dry substance to be hydrolyzed and that the rate of L-arabinose in the total amount of the acid-hydrolyzed monosaccharides is 50% or more.

In another preferred embodiment of the present invention, the acid-hydrolyzed solution is divided into the sections of a solution containing high amount of L-arabinose, xylooligosaccharide or galactooligosaccharide and insoluble residue.

Further, when the solution containing L-arabinose obtained in the above manufacturing process is hydrogenated, a sugar alcohol solution containing L-arabitol can be manufactured.

### Brief Description of the Drawing

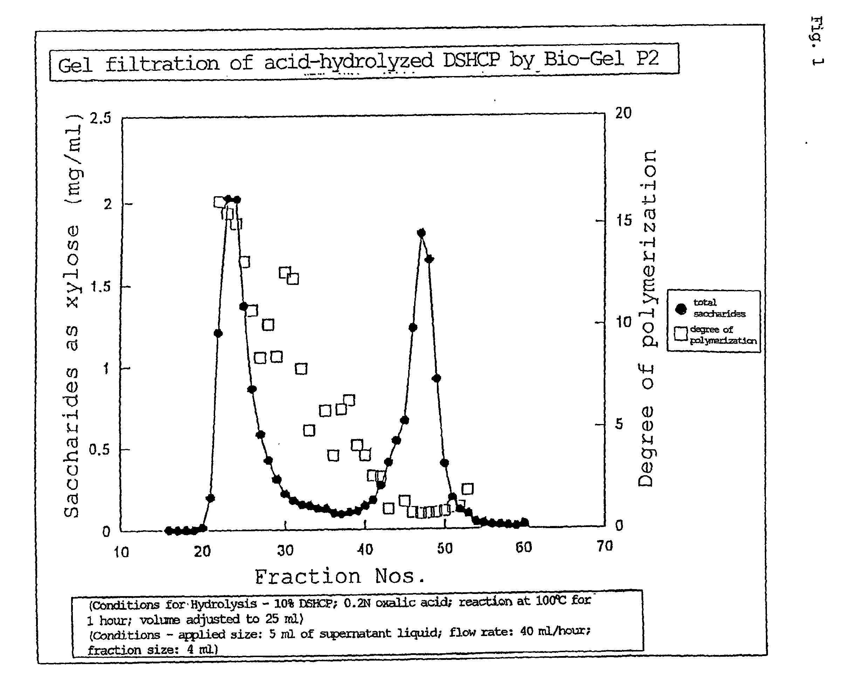

Fig. 1 shows gel filtration of the acid-hydrolyzed DSHCP in Bio-Gel P2.

### Embodiments of the Invention

The present invention will now be illustrated in detail as hereunder.

L-Arabinose in the vegetable fiber is present as arabinoxylan which is a polysaccharide and is a kind of a hemicellulose components in cell walls, particularly in envelopes of corn grains of plants of Gramineae, and is contained in an amount of about 23% as arabinose in the representative pure envelopes of corn grains and ear axis (Handbook of Dietary Fiber, Mark L. Dreher, Marcel Dekker, Inc., 1987; and Luc Saulnier, et al., Carbohydrate Polymers, 26, (1995), 279-287). It is reported that, in sugar beet fiber, it is present as arabinogalactan which is a polysaccharide and is a kind of a hemicellulose components and that the L-arabinose content in the sugar beet fiber is ~21% (V. Micard, et al., Carbohydrate Polymers, 32, (1997), 283-292). It is also reported that 55% of arabinose is present in a high-molecular pectin fraction in strained lees of apple juice (Henk A. Schols, et al., Carbohydrate Research, 206, (1990), 117-129). Accordingly, it is preferred that the vegetable fiber used contains L-arabinose as much as possible. If necessary, it is also possible to use a material wherefrom a part of or all of substances other than the vegetable fiber which are contaminated in the by-products such as starch, protein, lipid, dyes, etc. are removed. Further, the shape of the vegetable fiber may be in narrow stripes or in ground powder although the use of powdery thing is preferred.

With regard to the acid which is used in the present invention, it is able to lower the pH of the hydrolyzed solution. However, when the load upon purification of the acid-hydrolyzed solution by means of an ion-exchange resin is taken into consideration, it is preferred to use an acid which forms a salt being hardly soluble in the neutralization with a base after the acidic hydrolysis such as sulfuric acid which forms barium sulfate in the neutralization with barium hydroxide or oxalic acid which forms calcium oxalate in the neutralization with calcium hydroxide or with calcium oxide.

The procedure for the acidic hydrolysis according to the present invention is that, first, the acid is dissolved in cold or warm water. Concentration of the acid dissolved is adjusted to an extent of 0.01N to 0.50N or, preferably, 0.01N to 0.15N in terms of a normality.

Concentration of the acid used in the present invention is very important for liberation and production of L-arabinose from the vegetable fiber in high purity and yield.

When the acid concentration is 0.50N or higher, not only L-arabinose but also other saccharides such as D-xylose, D-galactose and D-glucose are liberated and produced together therewith whereupon the specificity of the acidic hydrolytic characteristics to L-arabinose is lost and, in addition, the L-arabinose which is once liberated and produced is further decomposed resulting in a cause to lower the content of L-arabinose. The acid concentration for an acidic hydrolysis of hemicellulose is usually 7% in case of sulfuric acid (corresponding to 1.5N) as shown, for example, in Example 1 of the Japanese Patent Laid-Open No. 312997/1989 and, in the present invention, there is a characteristic feature in that a specific acidic hydrolysis is carried out at the acid concentration of as low as 1/150 to 1/3 or, preferably, 1/150 to 1/10 of usual acid concentrations.

When the acid concentration is lower than 0.01N, the time required for the desired acidic hydrolysis is necessary to be long and that is not commercially preferred.

After that, vegetable fiber containing 10% (w/w) or more of L-arabinose component is added to an aqueous acid solution of the adjusted concentration so as to make its solid concentration 3% (w/w) to 20% (w/w) and fully suspended, the suspension is heated and the acidic hydrolysis is carried out under the state that the liquid temperature is kept constant at 80°C to 150°C or, preferably, at 96°C to 145°C.

When the solid concentration is less than 3% (w/w), concentration of the acid-hydrolyzed solution is low whereby a lot of energy is needed for the succeeding steps such as concentration and, therefore, that is not preferred. When the solid concentration is more than 20% (w/w), there is no fluidity after completion of the acidic hydrolysis whereby the handing becomes difficult in, for example, a filtering operation and, therefore, that is not preferred as well.

There is no particular limitation for a method of heating so far as a heating is possible by means of a direct heating (such as by an in-line heater of such a type where steam is directly blown in) or an indirect heating. With regard to vessel for the acidic hydrolysis, any of acid-resisting, heat-resisting and/or pressure-resisting and open or closed ones may be used and, preferably, a vessel having a structure which is capable of stirring the suspension is used. Reaction temperature is a factor which greatly affects the reaction rate. Basically, the rate of acidic hydrolysis is dependent upon the temperature and, therefore, the time required for the acidic hydrolysis becomes long at low temperature region and the higher the temperature range, the shorter the time therefor such as that, in general, the reaction rate is accelerated to an extent of about twice when the reaction temperature is raised to an extent of 10°C. When the temperature for the acidic hydrolysis is lower than 80°C, the reaction time becomes too long and the efficiency becomes bad while, when it is higher than 150°C, decomposition reaction of the liberated and produced saccharide becomes significant and coloration of the solution after completion of the acidic hydrolysis increases and, therefore, that is not preferred in view of economical reasons that decoloration and/or purifying load by ion-exchange resin increase(s).

Reaction time for the acidic hydrolysis may be free within a condition that the rate of the total amount of the liberated and eluted saccharides in the reaction solution to the vegetable fiber on the basis of dry substances (hereinafter, referred to as "solubilizing rate") is 30% or more and further that the rate of L-arabinose to the total amount of the monosaccharides in the reaction solution on the basis of dry substances (hereinafter, referred to as "occupying rate of L-arabinose") is 50% or more. When the " solubilizing rate" is less than 30%, the "occupying rate of L-arabinose" increases but the production yield lowers while, when the "occupying rate of L-arabinose" is less than 50%, the production yield increases but recovery of arabinose of high purity becomes difficult and, therefore, they are not preferred. Although the reaction depends upon the acid concentration and the hydrolyzing temperature for hydrolysis, it is usually able to finish within 10 minutes to 6 hours.

After that, the solution obtained by the acidic hydrolysis is subjected to a known separating process such as filtration through diatomaceous earth, centrifugation, filtration through membrane, filter press, etc. to remove decomposed residues and to make clear either as it is or after neutralizing the pH of the solution by adding to and mixing with a necessary amount of a base as a neutralizing agent by a conventional method.

With regard to a base as a neutralizing agent, it is preferred to use that which forms a hardly-soluble salt with an acid as mentioned already although there is no particular limitation therefor so far as it is a substance which is able to neutralize.

With regard to pH, it is also possible to stop at an isoelectric point for insolubilization of the eluate derived from protein contaminated in the solution and there is no particular limitation therefor.

It is also possible that, before the neutralized solution is filtered, the solution is cooled and/or filtered after being stored for a predetermined period with an object of full separation of hardly soluble salt and insoluble substances.

In addition, if necessary, the clarified saccharide solution may be subjected to a known method such as decolorizing purification and/or dialysis using bone carbon or activated carbon and purification by ion-exchange membrane, ion-exchange resin, etc. to give a solution containing both monosaccharide components where "occupying rate of L-arabinose" is 50% or more and useful xylooligosaccharide or galactooligosaccharide.

The solution containing a lot of useful saccharides prepared as such can be easily separated into two sections - a section of L-arabinose-rich solution and a section of functional xylooligosaccharide or galactooligosacchardie - by means of a known separating method (such as membrane filtration, gel filtration, chromatographic separation using activated carbon, etc.).

It is also possible to manufacture a sugar alcohol containing L-arabitol by hydrogenation of L-arabinose using a known method. It is further possible to manufacture an L-arabitol-rich solution by hydrogenation of the separated L-arabinose-rich solution.

### Examples

As hereunder, the present invention will be illustrated by way of Examples although the technical coverage of the present invention is not limited by the said Examples. The vegetable fiber used for obtaining the detailed data was envelope of corn grains prepared by a complete removal of starch from the fiber obtained by a wet process for manufacturing the starch. With regard to the component composition such as total arabinose content in the envelope of corn grains and the structure of arabinoxylan in hemicellulose, a report by Luc Saulnier, et al. (Carbohydrate Polymers, 26(1995), 279-287) was referred to. Incidentally, sugar content was measured by a phenyl-sulfuric acid method, sugar composition was measured by an HPLC and reducing sugar was measured by a Somogyi-Nelson method and the hydrolyzing rates of L-arabinose and D-xylose were measured in terms of the rate of liberated and produced L-arabinose to the total arabinose content in the envelope and of the rate of liberate/produced D-xylose to the total xylose content therein, respectively.

### Example 1.

Envelopes of corn grains (1.0 g in terms of dry weight) were added to a 15-ml vial equipped with a screw cap, 10 ml of 0.05N~0.50N sulfuric acid solution were added, the vial was tightly sealed and hydrolysis was carried out in boiling water for 1 hour. To the acid-hydrolyzed solution was added barium hydroxide necessary for neutralization of sulfuric acid followed by shaking in a refrigerator. This was made 25 ml and envelopes of corn grains which were not acid-hydrolyzed and barium sulfate were removed by a centrifuge to give a solution containing useful saccharides. Result of measurement of solubilizing rate, saccharide content, hydrolyzing rate and occupying rate of L-arabinose for the solution is given in Table 1. The useful saccharides produced from the envelopes of corn grains are given in Table 1 in terms of the producing rate from the envelopes of corn grains.

**Table 1**

| Influence of Sulfuric Acid Concentration on Production of Functional Oligosaccharide and Functional Monosaccharides from Envelopes of Corn Grains when Sulfuric Acid was Used | | | | | | | |
|---|---|---|---|---|---|---|---|
| Sulfuric Acid Concentration (N) | Solubilizing Rate (%) | L-Arabinose (%) | D-Xylose | Xylooligo-saccharide (%) | Hydrolyzing Rate (%) | | Occupying Rate of L-Arabinose (%) |
| | | | | | L-Arabinose | D-Xylose | |
| 0.05 | 13.0 | 5.0 | 0.4 | 7.6 | 22 | 1 | 93 |
| 0.10 | 28.4 | 10.8 | 2.1 | 15.5 | 47 | 6 | 84 |
| 0.15 | 35.2 | 11.7 | 4.6 | 18.9 | 51 | 13 | 71 |
| 0.50 | 44.7 | 14.1 | 20.0 | 10.6 | 62 | 58 | 38 |

### Example 2.

Envelopes of corn grains (1.0 g in terms of dry weight) were added to a 15-ml vial equipped with a screw cap, 10 ml of 0.01N~0.50N hydrochloric acid solution were added, the vial was tightly sealed and hydrolysis was carried out in boiling water for 1 hour. To the acid-hydrolyzed solution was added sodium hydroxide necessary for neutralization of hydrochloric acid followed by shaking in a refrigerator. This was made 25 ml and envelopes of corn grains which were not acid-hydrolyzed was removed by a centrifuge to give a solution containing useful saccharides. Result of measurement of solubilizing rate, saccharide content, hydrolyzing rate and occupying rate of L-arabinose for the solution is given in Table 2. The useful saccharides produced from the envelopes of corn grains are given in Table 2 in terms of the producing rate from the envelopes of corn grains.

**Table 2**

| Influence of Hydrochloric acid Concentration on Production of Functional Oligosaccharide and Functional Monosaccharides from Envelopes of Corn Grains when Hydrochloric acid was Used | | | | | | | |
|---|---|---|---|---|---|---|---|
| Hydro-chloric acid Concentration (N) | Solubilizing Rate (%) | L-Arabinose (%) | D-Xylose (%) | Xylooligo-saccharide (%) | Hydrolyzing Rate (%) | | Occupying Rate of L-Arabinose (%) |
| | | | | | L-Arabinose | D-Xylose | |
| 0.01 | 2.9 | 0.4 | 0.1 | 2.4 | 2 | 0 | 88 |
| 0.05 | 41.3 | 9.8 | 3.3 | 28.2 | 43 | 10 | 75 |
| 0.10 | 57.1 | 11.7 | 11.3 | 34.1 | 51 | 33 | 50 |
| 0.50 | 53.5 | 13.9 | 30.9 | 1.9 | 61 | 90 | 27 |

### Example 3.

Envelopes of corn grains (1.0 g in terms of dry weight) were added to a 15-ml vial equipped with a screw cap, 10 ml of 0.05N~2.00N oxalic acid solution were added, the vial was tightly sealed and hydrolysis was carried out in boiling water for 1 hour. To the acid-hydrolyzed solution was added calcium oxide necessary for neutralization of oxalic acid followed by shaking in a refrigerator. This was made 25 ml and envelopes of corn grains which were not acid-hydrolyzed and calcium oxalate were removed by a centrifuge to give a solution containing useful saccharides. Result of measurement of solubilizing rate, saccharide content, hydrolyzing rate and occupying rate of L-arabinose for the solution is given in Table 3. The useful saccharides produced from the envelopes of corn grains are given in Table 3 in terms of the producing rate from the envelopes of corn grains. Incidentally, Fig. 1 shows the result of gel filtration using Bio-Gel by P2 for the product made to react at 100°C for 1 hour using 0.20N oxalic acid.

**Table 3**

| Influence of Oxalic acid Concentration on Production of Functional Oligosaccharide and Functional Monosaccharides from Envelopes of Corn Grains when Oxalic acid was Used | | | | | | | |
|---|---|---|---|---|---|---|---|
| Oxalic acid Concentration (N) | Solubilizing Rate (%) | L-Arabinose (%) | D-Xylose (%) | Xylooligo-saccharide (%) | Hydrolyzing Rate (%) | | Occupying Rate of L-Arabinose (%) |
| | | | | | L-Arabinose | D-Xylose | |
| 0.05 | 12.1 | 2.9 | 0.3 | 8.9 | 13 | 1 | 91 |
| 0.10 | 28.0 | 8.6 | 1.2 | 18.2 | 38 | 3 | 88 |
| 0.20 | 47.9 | 12.7 | 3.3 | 31.7 | 56 | 10 | 78 |
| 0.50 | 62.8 | 13.2 | 7.0 | 42.1 | 58 | 20 | 64 |
| 1.00 | 62.9 | 15.3 | 12.3 | 34.3 | 67 | 36 | 53 |
| 2.00 | 39.9 | 16.8 | 21.6 | 0.0 | 74 | 63 | 42 |

### Example 4.

Envelopes of corn grains (1.0 g in terms of dry weight) were added to a 15-ml vial equipped with a screw cap, 10 ml of 0.1N sulfuric acid solution were added, the vial was tightly sealed and hydrolysis was carried out in boiling water for 0.5 to 6.0 hour(s). To the acid-hydrolyzed solution was added barium hydroxide necessary for neutralization of sulfuric acid followed by shaking in a refrigerator. This was made 25 ml and envelopes of corn grains which were not acid-hydrolyzed was removed by a centrifuge to give a solution containing useful saccharides. Result of measurement of solubilizing rate, saccharide content, hydrolyzing rate and occupying rate of L-arabinose for the solution is given in Table 4. The useful saccharides produced from the envelopes of corn grains are given in Table 4 in terms of the producing rate from the envelopes of corn grains.

**Table 4**

| Influence of Acid-Hydrolyzing Time on Production of Functional Oligosaccharide and Functional Monosaccharides from Envelopes of Corn Grains when 0.1N Sulfuric Acid was Used | | | | | | | |
|---|---|---|---|---|---|---|---|
| Acid-Hydrolyzing Time (hours) | Solubilizing Rate (%) | L-Arabinose (%) | D-Xylose (%) | Xylooligo-saccharide (%) | Hydrolyzing Rate (%) | | Occupying Rate of L-Arabinose (%) |
| | | | | | L-Arabinose | D-Xylose | |
| 0.5 | 30.7 | 7.9 | 1.5 | 21.3 | 35 | 4 | 84 |
| 1.0 | 30.7 | 10.8 | 3.1 | 16.8 | 47 | 9 | 69 |
| 2.0 | 60.9 | 11.8 | 8.4 | 40.7 | 52 | 24 | 58 |
| 3.0 | 60.4 | 12.3 | 11.9 | 36.2 | 54 | 35 | 50 |
| 6.0 | 62.7 | 12.5 | 19.4 | 30.8 | 55 | 57 | 38 |

### Example 5.

Envelopes of corn grains (1.0 g in terms of dry weight) were added to a 15-ml vial equipped with a screw cap, 10 ml of 0.05N hydrochloric acid solution were added, the vial was tightly sealed and hydrolysis was carried out in boiling water for 0.5 to 6.0 hour(s). To the acid-hydrolyzed solution was added sodium hydroxide necessary for neutralization of hydrochloric acid followed by shaking in a refrigerator. This was made 25 ml and envelopes of corn grains which were not hydrolyzed was removed by a centrifuge to give a solution containing useful saccharides. Result of measurement of solubilizing rate, saccharide content, hydrolyzing rate and occupying rate of L-arabinose for the solution is given in Table 5. The useful saccharides produced from the envelopes of corn grains are given in Table 5 in terms of the producing rate from the envelopes of corn grains.

**Table 5**

| Influence of Acid-Hydrolyzing Time on Production of Functional Oligosaccharide and Functional Monosaccharides from Envelopes of Corn Grains when 0.05N Hydrochloric Acid was Used | | | | | | | |
|---|---|---|---|---|---|---|---|
| Acid-Hydrolyzing Time (hours) | Solubilizing Rate (%) | L-Arabinose (%) | D-Xylose (%) | Xylooligo-saccharide (%) | Hydrolyzing Rate (%) | | Occupying Rate of L-Arabinose (%) |
| | | | | | L-Arabinose | D-Xylose | |
| 0.5 | 30.8 | 6.3 | 2.1 | 22.4 | 28 | 6 | 75 |
| 1.0 | 40.9 | 7.1 | 3.3 | 30.5 | 31 | 10 | 69 |
| 2.0 | 62.4 | 10.5 | 8.0 | 43.2 | 46 | 23 | 55 |
| 3.0 | 62.4 | 11.0 | 10.6 | 39.8 | 48 | 31 | 49 |
| 6.0 | 60.8 | 9.7 | 14.6 | 35.0 | 43 | 43 | 38 |

### Example 6.

Envelopes of corn grains (1.0 g in terms of dry weight) were added to a 15-ml vial equipped with a screw cap, 10 ml of 0.1N oxalic acid solution were added, the vial was tightly sealed and hydrolysis was carried out in boiling water for 0.5 to 6.0 hour(s). To the acid-hydrolyzed solution was added calcium oxide necessary for neutralization of oxalic acid followed by shaking in a refrigerator. This was made 25 ml and envelopes of corn grains which were not acid-hydrolyzed was removed by a centrifuge to give a solution containing useful saccharides. Result of measurement of solubilizing rate, saccharide content, hydrolyzing rate and occupying rate of L-arabinose for the solution is given in Table 6. The useful saccharides produced from the envelopes of corn grains are given in Table 6 in terms of the producing rate from the envelopes of corn grains.

**Table 6**

| Influence of Acid-Hydrolyzing Time on Production of Functional Oligosaccharide and Functional Monosaccharides from Envelopes of Corn Grains when 0.1N Oxalic Acid was Used | | | | | | | |
|---|---|---|---|---|---|---|---|
| Acid-Hydrolyzing Time (hours) | Solubilizing Rate (%) | L-Arabinose (%) | D-Xylose (%) | Xylooligo-saccharide (%) | Hydrolyzinc Rate (%) | | Occupying Rate of L-Arabinose (%) |
| | | | | | L-Arabinose | D-Xylose | |
| 0.5 | 21.6 | 5.4 | 0.6 | 15.6 | 24 | 2 | 90 |
| 1.0 | 27.8 | 7.8 | 1.1 | 18.9 | 34 | 3 | 88 |
| 2.0 | 44.8 | 11.3 | 3.1 | 30.4 | 50 | 9 | 78 |
| 3.0 | 56.8 | 12.2 | 5.7 | 38.9 | 54 | 17 | 68 |
| 6.0 | 51.3 | 12.2 | 5.9 | 33.2 | 54 | 17 | 68 |

### Example 7.

Envelopes of corn grains(500 g in terms of dry weight) were added to a tightly-closing autoclave equipped with a stirring jacket (210 mm diameter and 175 mm height), 4,500 g of 0.022N oxalic acid solution were added thereto, the liquid temperature was raised to 130°C by means of both direct and indirect heating using steam (vapor pressure: 4.0 kg/cm²) with stirring and tight closing and, after that, the temperature was maintained at 130°C by means of a direct heating only whereupon an acidic hydrolysis was carried out. During that period, an autoclave (volume: 100 ml) equipped with a condenser was used intermittently for preventing an increase in concentration due to flushing of the reaction solution, a sampling was carried out and, after completion of the reaction, calcium hydroxide was added to 100 g of the reaction solution to neutralize. Water was added to the neutralized solution to make the total amount 200 g and the envelopes which were not acid-hydrolyzed and calcium oxalate were removed by a centrifuge to give a solution containing useful saccharides. Result of measurement of solubilizing rate, saccharide content, hydrolyzing rate and occupying rate of L-arabinose for the solution is given in Table 7. Incidentally, with regard to the amount of water coming thereinto by a direct heating with steam, an increase in weight in the solution after completion of all reactions as compared with the liquid weight before the reaction was applied. The useful saccharides produced from the envelopes of corn grains are given in Table 7 in terms of the producing rate from the envelopes of corn grains.

**Table 7**

| Influence of Acid-Hydrolyzing Time on Production of Functional Oligosaccharide and Functional Monosaccharides from Envelopes of Corn Grains when 0.022N Oxalic Acid was Used | | | | | | | |
|---|---|---|---|---|---|---|---|
| Acid-Hydrolyzing Time (minutes) | Solubilizing Rate (%) | L-Arabinose (%) | D-Xylose (%) | Xylooligo-saccharide (%) | Hydrolyzing Rate (%) | | Occupying Rate of L-Arabinose (%) |
| | | | | | L-Arabinose | D-Xylose | |
| 15 | 28.3 | 5.0 | 0.7 | 22.7 | 22 | 2 | 88 |
| 30 | 33.7 | 8.1 | 1.1 | 24.5 | 36 | 3 | 88 |
| 60 | 42.3 | 11.9 | 1.8 | 28.6 | 52 | 5 | 86 |
| 90 | 48.1 | 14.2 | 2.7 | 31.2 | 62 | 8 | 84 |
| 120 | 52.9 | 15.9 | 3.4 | 33.6 | 70 | 10 | 82 |
| 150 | 54.8 | 16.6 | 4.0 | 34.2 | 73 | 12 | 81 |
| 180 | 56.8 | 16.0 | 4.4 | 36.4 | 70 | 13 | 79 |

### Advantage of the Invention

In accordance with the present invention, there is now provided, by the use of very mild acid-hydrolyzing conditions which have not been thought of before, a commercial process for the manufacture of useful saccharides such as L-arabinose and oligosaccharides which are very useful in view of physiological function in a specific manner in high purity and also in large quantities from L-arabinose-containing vegetable fiber which is produced as a by-product from the manufacturing steps in various milling factories and starch factories.

## Claims

1. A process for the manufacture of L-arabinose, **characterized in that** vegetable fiber which is a by-product in the manufacture of corn starch, such as envelopes of corn grains, axis of ear of corn, wheat bran, barley bran, oat bran, rye bran, rice bran or defatted rice bran, is contacted with sulfuric acid, hydrochloric acid or oxalic acid, an acidic hydrolysis is carried out under conditions such that i) the concentration of acid is from 0.01 N to 0.15N for sulfuric acid or hydrochloric acid, and from 0.01 N to 0.50N for oxalic acid, and ii) the proportion of L-arabinose in the total amount of the acid-hydrolyzed monosaccharides is 50% or more, whereby L-arabinose contained in the vegetable fiber is selectively produced.

2. The process for the manufacture of L-arabinose according to Claim 1, **characterized in that** the vegetable fiber used contains 10% or more of at least L-arabinose as a part of the constituting saccharides on the basis of the dried vegetable fiber.

3. The process for the manufacture of L-arabinose according to Claim 1 or Claim 2, **characterized in that** the acidic hydrolysis is carried out under conditions such that the solid concentration of the vegetable fiber is from 3% (w/w) to 20% (w/w).

4. The process for the manufacture of L-arabinose according to any of Claims 1 to 3, **characterized in that** the acidic hydrolysis is carried out under conditions such that the temperature is from 80°C to 150°C.

5. The process for the manufacture of L-arabinose according to any of Claims 1 to 4, **characterized in that** the acidic hydrolysis is carried out under conditions such that the total amount of the saccharides decomposed and eluted during the acidic hydrolysis is 30% or more on the basis of the dry substance to be hydrolyzed.

6. The process for the manufacture of L-arabinose according to any of Claims 1 to 5, **characterized in that** the acid-hydrolyzed solution is divided into the parts of the solution containing a high amount of L-arabinose, xylooligosaccharide or galactooligosaccharide and insoluble residue.

## Patentansprüche

1. Verfahren zur Herstellung von L-Arabinose, **dadurch gekennzeichnet, dass** Pflanzenfasern, die ein Nebenprodukt bei der Herstellung von Getreidestärke sind, wie Hüllen von Getreidekörnern, Getreideährenspindel, Weizenkleie, Gerstenkleie, Haferkleie, Roggenkleie, Reiskleie oder entfettete Reiskleie, mit Schwefelsäure, Salzsäure oder Oxalsäure in Kontakt gebracht werden, eine saure Hydrolyse unter solchen Bedingungen durchgeführt wird, dass i) die Konzentration an Säure zwischen 0,01 N und 0,15 N bei Schwefelsäure oder Salzsäure und zwischen 0,01 N und 0,50 N bei Oxalsäure liegt und ii) der Anteil an L-Arabinose in der Gesamtmenge der säurehydrolysierten Monosaccharide 50 % oder mehr beträgt, wodurch in den Pflanzenfasern enthaltene L-Arabinose selektiv gewonnen wird.

2. Verfahren zur Erstellung von L-Arabinose nach Anspruch 1, **dadurch gekennzeichnet, dass** die verwendeten Pflanzenfasern 10 % oder mehr mindestens von L-Arabinose als Teil der Saccharidbestandteile bezogen auf die getrockneten Pflanzenfasern enthalten.

3. Verfahren zur Herstellung von L-Arabinose nach Anspruch 1 oder Anspruch 2, **dadurch gekennzeichnet, dass** die saure Hydrolyse unter solchen Bedingungen durchgeführt wird, dass die Feststoffkonzentration der Pflanzenfasern zwischen 3 Gew.-% und 20 Gew.-% beträgt.

4. Verfahren zur Herstellung von L-Arabinose nach einem der Ansprüche 1 bis 3, **dadurch gekennzeichnet, dass** die saure Hydrolyse unter solchen Bedingungen durchgeführt wird, dass die Temperatur von 80 °C bis 150 °C beträgt.

5. Verfahren zur Herstellung von L-Arabinose nach einem der Ansprüche 1 bis 4, **dadurch gekennzeichnet, dass** die saure Hydrolyse unter solchen Bedingungen durchgeführt wird, dass die Gesamtmenge an bei der sauren Hydrolyse zersetzten und eluierten Sacchariden 30 % oder mehr bezogen auf die zu hydrolysierende Trockensubstanz beträgt.

6. Verfahren zur Herstellung von L-Arabinose nach einem der Ansprüche 1 bis 5, **dadurch gekennzeichnet, dass** die säurehydrolysierte Lösung in die Teile der Lösung, die eine hohe Menge an L-Arabinose, Xylooligosaccharid oder Galacotooligosaccharid enthalten, und unlöslichen Rückstand aufgeteilt wird.

## Revendications

1. Procédé pour la production de L-arabinose, **caractérisé en ce qu'**une fibre végétale qui est un sous-produit de la production de l'amidon de maïs, tel que des enveloppes de grains de maïs, l'axe de l'épi du maïs, le son de blé, le son d'orge, le son d'avoine, le son de seigle, le son de riz ou le son de riz dégraissé, est mise en contact avec de l'acide sulfurique, de l'acide chlorhydrique ou de l'acide oxalique, une hydrolyse acide est effectuée dans des conditions telles que i) la concentration d'acide est de 0,01 N à 0,15 N pour l'acide sulfurique ou l'acide chlorhydrique, et de 0,01 N à 0,50 N pour l'acide oxalique, et ii) la proportion de L-arabinose dans la quantité totale des monosaccharides ayant subi l'hydrolyse acide est égale ou supérieure à 50 %, ce qui fait que le L-arabinose présent dans la fibre végétale est produit sélectivement.

2. Procédé pour la production de L-arabinose suivant la revendication 1, **caractérisé en ce que** la fibre végétale utilisée contient 10 % ou plus d'au moins L-arabinose comme partie des saccharides constitutifs sur la base de la fibre végétale déshydratée.

3. Procédé pour la production de L-arabinose suivant la revendication 1 ou la revendication 2, **caractérisé en ce que** l'hydrolyse acide est effectuée dans des conditions telles que la concentration en matières solides dans la fibre végétale est de 3 % (en poids/poids) à 20 % (en poids/poids).

4. Procédé pour la production de L-arabinose suivant l'une quelconque des revendications 1 à 3, **caractérisé en ce que** l'hydrolyse acide est effectuée dans les conditions telles que la température est de 80°C à 150°C.

5. Procédé pour la production de L-arabinose suivant l'une quelconque des revendications 1 à 4, **caractérisé en ce que** l'hydrolyse acide est effectuée dans les conditions telles que la quantité totale des saccharides décomposés et élués au cours de l'hydrolyse acide est égale ou supérieure à 30 % sur la base de la substance sèche à hydrolyser.

6. Procédé pour la production de L-arabinose suivant l'une quelconque des revendications 1 à 5, **caractérisé en ce que** la solution ayant subi l'hydrolyse acide est divisée en parties consistant en la solution contenant une grande quantité de L-arabinose, de xylo-oligosaccharide ou galactooligosaccharide et un résidu insoluble.
